Europäisches Patentamt

European Patent Office

Office européen des brevets

(19)

(11) Publication number: **0 074 082**
**B1**

(12) **EUROPEAN PATENT SPECIFICATION**

(45) Date of publication of patent specification: **21.10.87**

(51) Int. Cl.⁴: **A 61 K 7/18,** A 61 K 31/315

(21) Application number: **82108052.0**

(22) Date of filing: **01.09.82**

(54) **Stable oral compositions containing zinc and fluoride compounds.**

(30) Priority: **03.09.81 US 299046**

(43) Date of publication of application:
**16.03.83 Bulletin 83/11**

(45) Publication of the grant of the patent:
**21.10.87 Bulletin 87/43**

(84) Designated Contracting States:
**AT BE CH DE FR GB IT LI LU NL SE**

(56) References cited:
**EP-A-0 075 446**
**US-A-4 022 880**
**US-A-4 138 477**
**US-A-4 152 418**

(73) Proprietor: **Richardson-Vicks, Inc.**
**Ten Westport Road**
**Wilton, CT 06897 (US)**

(72) Inventor: **Shah, Nutan B.**
**160 Berrian Road**
**New Rochelle New York 10804 (US)**
Inventor: **Schmidt, Nicholas F.**
**11 Arapaho Drive**
**Brookfield Connecticut 06850 (US)**

(74) Representative: **Vossius & Partner**
**Siebertstrasse 4 P.O. Box 86 07 67**
**D-8000 München 86 (DE)**

The file contains technical information submitted after the application was filed and not included in this specification

Courier Press, Leamington Spa, England.

# 0 074 082

**Description**

Field of the invention
This invention relates to stable water-containing oral compositions containing zinc and fluoride.

Background of the invention
The prior art is replete with oral compositions containing zinc salts for various beneficial effects believed to be imparted by the zinc ion, for example, reduction of calculus formation or inhibition of offensive mouth odor due to fermentation and putrefaction occurring in the oral cavity. However, despite the heretofore known use of zinc compounds in dental compositions, their use has not been without certain drawbacks. For example, when such soluble zinc compounds have been employed, it has not been possible to satisfactorily incorporate a soluble ionic fluoride salt, such as an alkali metal, ammonium and stannous fluorides, for anti-caries activity in the compositions due to the inherent chemical incompatability therebetween in the presence of water. This is generally evidenced by the precipitation of insoluble fluoride with a resultant descrease in the amount of efficacious fluoride available in the composition.

In US—A—2,527,686, for example, a mouthwash is disclosed containing soluble zinc chloride, soluble ionic fluoride and, in addition, formaldehyde and two ferments, papain and malt. However, the zinc/fluoride combination is not stable in such a formulation as evidenced by undesirable cloudiness and precipitation of insoluble by-products.

Attempts have thus been made either to use insoluble or sparingly soluble zinc salts or to utilize zinc which has been chemically modified in some manner, for example, by interaction with other adjuvants in order to decrease its potential for forming an insoluble by-product in the presence of ionic fluoride, such as by complexation with a zinc complexing agent, or by addition of a solvating agent to effect solvation of the zinc salt. For example, in US—A—4,138,477, oral compositions are disclosed containing a zinc-polymer complex formed by the reaction of a zinc salt and an anionic polymer; in copending US—A—4,325,939 entitled "Zinc Derivatives and Their Use in Dental Compositions", an alkali metal or ammonium zinc citrate complex is described; and in copending US—A—4,289,755 entitled "Stable Mouthwash Compositions Containing Zinc and Fluoride Compounds", the solvation of zinc citrate by the addition of citric acid is described.

Either or both of the zinc and fluoride salts may also be physically modified in order to lessen the problem of zinc/fluoride incompatibility, for example, by use of well known sustained release or microencapsulation physical forms which allow permeation of the particular salt from protective particles into the oral composition over a period of time, thereby reducing the rate of undesirable interaction between the two salts. For example, in US—A—4,220,522, microencapsulation of sodium fluoride by a particular pretreated form of a lower alkyl cellulose is disclosed for use in dental formulations.

It is therefore highly desirable to provide a stable oral composition containing a water soluble zinc salt capable of preventing and controlling mouth odor but which does not present any substantial chemical incompatibility when admixed with an ionic fluoride salt in the presence of water. It is also desirable that said zinc salt and said fluoride salt be utilized as such without the need for either or both being modified by chemical or physical means.

Brief description of the invention
In accordance with the invention, a stable water-containing oral composition being essentially free of $ZnF_2$ precipitant is provided comprising a semi-solid dentifrice vehicle as an oral vehicle, a water soluble organic zinc salt and a soluble ionic fluoride salt, said zinc salt and said fluoride salt being essentially unmodified in situ by additional chemical or physical agents.

Detailed description of the invention
Provided herewith are stable water-containing oral formulations comprising a combination of a water soluble organic zinc salt selected from zinc salicylate (preferred), zinc lactate and zinc gluconate, including mixtures thereof, as an effective agent against mouth odor and an inorganic water soluble ionic fluoride salt as a source of soluble fluorine for effective anti-caries activity. Typical such ionic fluorides include ammonium fluoride and alkali metal, alkaline earth metal and heavy metal fluoride salts, e.g., sodium fluoride, potassium fluoride, lithium fluoride, stannous fluoride, stannic fluoride and barium fluoride, ionic fluoride salts generally acceptable in oral formulations as a source of soluble fluorine. Ammonium fluoride, alkali metal and tin fluorides, particularly sodium and stannous fluorides, respectively, are preferred.

Surprisingly, no significant incompatability is observed between the three aforementioned zinc salts and said ionic fluorides in aqueous solution so that the need heretofore for chemically or physically modifying the zinc and/or fluoride components by additional adjuvants which inhibit the interaction between zinc and fluoride is eliminated.

Accordingly, the oral compositions of this invention comprise a semi-solid dentifrice vehicle as an oral vehicle, an effective amount for controlling mouth odor of a zinc salt selected from zinc salicylate, zinc lactate, zinc gluconate and mixtures thereof and an effective anti-caries amount of a soluble ionic fluoride salt, said zinc salt and said fluoride salt being in situ in essentially free form. As the oral vehicle, any conventional semi-solid dentifrice may be utilized.

2

In the subject compositions, the preferred amount of zinc salt employed is sufficient to provide from 0.01 to 5.0 weight percent of theoretical zinc element, more preferably from 0.05 to 2.0 weight percent of elemental zinc, and most preferably from 0.08 to 1.0 weight percent of elemental zinc. As used herein, the term "weight percent" denotes either weight/weight (w/w) or weight/volume (w/v) percent, i.e., the weight percent of elemental zinc based on the total weight or total volume (i.e., 100 percent) of the final composition depending on whether it is a solid or liquid, respectively. In the preferred liquid embodiments utilizing zinc salicylate as the preferred zinc salt, for example, the amount of zinc salicylate is preferably from 0.05 to 25.0 w/v percent, more preferably from 0.25 to 10.0 w/v percent, and most preferably from 0.4 to 5.0 w/v percent.

The mount of ionic fluoride salt preferably employed in the subject compositions is an amount sufficient to provide from 25 to 15000 parts per million (ppm) of available fluoride ion, more preferably from 100 to 3000 ppm of available fluoride ion, and most preferably from 200 to 1000 ppm of available fluoride ion, again based on the total weight or volume of the final composition depending on whether it is a solid or liquid, respectively.

As used herein, the term "oral composition" means a product which in the ordinary course of usage is not intentionally swallowed for purposes of systemic administration of the particular therapeutically active ingredient(s), but is retained in the oral cavity for a time sufficient to contact substantially all of the dental surfaces and/or oral tissues for purposes of local activity. Typical oral compositions for purposes of the present invention are those containing at least an amount of water sufficient to solubilize the zinc and fluoride components. The oral compositions are semi-solid dentifrices such as dental creams, pastes and gels, all of which compositions contain substantial amounts of water, generally far more than enough to maintain the specified amount of said zinc and fluoride salts in solution.

The present invention thus provides a water-containing oral composition comprising an effective mouth odor inhibiting amount of an organic zinc salt selected from zinc salicylate, zinc lactate, zinc gluconate and mixtures thereof, an effective anti-caries amount of a water-soluble inorganic fluoride salt, said zinc salt and said fluoride salt being in situ essentially unmodified by additional chemical or physical means, and a semi-solid dentifrice vehicle as an oral vehicle containing at least sufficient water to solubilize said zinc salt and said fluoride salt.

It is understood, however, that it is within the broader aspect of the invention to include other oral compositions with oral vehicles such as lozenges and chewing gums wherein water may be present during their manufacture but little if any when finished. In certain sugarless gums, for example, there can be used as the binder ingredient a solution of sorbitol in water containing from 10% to 80%, preferably from 50% to 75% by weight of the sorbitol in water. In others, there is used a gum acacia-in-water system containing from 30% to 60%, preferably from 45% to 50%, by weight of gum acacia powder in water. Similarly, lozenge formulations may contain water in aqueous systems during formation of the lozenge mass. Incorporation of the zinc salt and ionic fluoride into such formulations may readily be accomplished without fear of precipitating insoluble fluorides by simply utilizing an appropriately prepared aqueous solution of the zinc and fluoride components. After incorporation, drying of the oral composition, as in the case of lozenges, is then performed to remove all or part of the moisture.

As noted previously, it is surprising that the three selected zince salts and said ionic fluorides are compatible and remain simply in such compositions without being chemically or physically modified by additional adjuvants.

The heretofore mentioned oral formulations are exemplary only. Many additional water-containing oral formulations are described in the prior art and, in carrying out this invention, such formulations can be employed so long as there is at least sufficient water to solubilize both the zinc and fluoride components either in the final product or during the course of their manufacture.

The subject oral compositions are generally prepared in accordance with art-recogr 'zed practices. Since, however, the two essential components of this invention are the specified zinc salt and the ionic fluoride, it is obvious that any other ingredient in the oral compositions must not interact with either or both or these two components so as to substantially lessen their therapeutic effectiveness. For example, dental cream abrasives or additives which contain soluble phosphates should be avoided due to the interaction with zinc and abrasives which contain calcium also should be avoided due to the interaction with fluoride. In general, therefore, any conventional ingredients can be used so long as they are compatible, i.e., do not interfere with the activity of the zinc or fluoride component.

Since the problem of zinc/fluoride incompatibility is obviated by the present invention, the subject compositions can be prepared without undue concern for the order or manner of incorporating the two essential components.

In the substantially solid or semi-solid oral compositions of this invention, such as dental creams, pastes and gels, the liquids and solids should be proportioned to form an extrudable creamy mass of desirable consistency. In general, liquids in these formulations will comprise chiefly water, glycerin, sorbitol or propylene glycol including suitable mixtures thereof. It is advantageous usually to use a mixture of both water and a humectant or binder such a glycerin or sorbitol, preferably glycerin. The total liquid content will generally be 20 to 75 percent by weight of the formulation. It is also preferred to use a gelling agent such as a natural or synthetic gum and gum-like material, e.g. Irish moss, gum traganth, xanthan gum, Veegum regular, sodium carboxymethylcellulose, polyvinylpyrrolidone and starch. The Irish moss

3

0 074 082

and sodium carboxymethylcellulose are compatible particularly and are preferred gelling agents. The gum content is usually in an amount up to 10 percent and preferably 0.5 to 5 percent by weight of the formulation.

An essential ingredient in dental cream formulations is an effective abrasive amount of a suitable dental abrasive, generally from 10 to 60 percent by weight and preferably from 20 to 50 percent by weight. As noted previously, this abrasive must not interact with either the zinc or fluoride component. Typical compatible abrasives include, for example, insoluble metalphosphates, finely divided silicas and bentonite. The preferred abrasive is silica.

Various other materials may be incorporated as adjuvants in dental creams. Examples thereof are coloring or whitening agents, preservatives, silicones, chlorophyll compounds, ammoniated materials such as urea, diammoniumphosphate and mixtures thereof. A small amount of colloidal silica, for example, is often incorporated into toothpaste formulations as a thickener, giving some body to the formulation upon swelling when in contact with water. The foregoing adjuvants are suitably selected and incorporated in the instant compositions in amounts which do not substantially adversely affect the properties and characteristics desired for the particular type of composition.

For some purposes it may be desirable to include antibacterial agents in the dental creams. Typical antibacterial agents which may be used in amounts of 0.01 percent to 5 percent, preferably 0.05 percent to 1.0 percent, by weight of the composition include: $N^1$ - (4 - chlorobenzyl) - $N^5$ - (2,4 - dichlorobenzyl)-biguanide, p - chlorophenyl biguanide, 4 - chlorobenzyhydryl biguanide, 4 - chlorobenzhydrylguanylurea, N - 3 - lauroxpropyl - $N^5$ - p - chlorobenzylbiguanide, 1,6 - di - p - chlorophenylbiguanidohexane, 1 - (lauryldimethylammonium) - 8 - (p - chlorobenzyldimethlyammonium)octane dichloride, 5,6 - dichloro - 2 - quanidinobenzimidazole, $N^1$ - p - chlorophenyl - $N^5$ - laurylbiguanide, 5 - amino - 1,3 - bis(2 - ethylenyl) - 5 - methylhexahydropyrimidine, and their non-toxic acid addition salts.

Tooth desensitization agents such as, for example, a nitrate of potassium, lithium or sodium disclosed in US—A—3,863,006 may also be incorporated in tooth desensitizing amounts, generally up to 20% and preferably 5% by weight.

Any suitable flavoring or sweetening materials may also be employed. Examples of suitable flavoring constituents include the flavoring oils, e.g. oils of spearmint, peppermint, wintergreen, sassafras, clove, sage, eucalyptus, marjoram, cinnamon, lemon and orange, as well as sodium methylsalicylate. Suitable natural and synthetic sweetening agents include sucrose, lactose, maltose, sorbitol, sodium cyclamate, ammonium glycyrrhizinate and its derivatives and saccharin. Suitably, flavor and sweetening agents may together comprise from 0.01 to 5 percent or more by weight of the dental cream.

The dental cream should have a pH practicable for use. An acidic to neutral pH from 3.0 to 7.0 is preferred with 4.0 to 6.5 most preferred. An appropriate acidic buffer may be used to stabilize the pH, for example, an acid/salt buffer such as citric acid/citrate, malic acid/malate and adipic acid/adipate. Particularly useful when zinc salicylate is employed as the zinc salt component is a buffer of salicylic acid/sodium salicylate. The pH determination is made on a 10% aqueous suspension of the dentifrice. If necessary, conventional acidic materials may be added to adjust the pH as desired.

When visually clear gels are employed, polishing agents comprising alkali metal aluminosilicate complexes are particularly useful, since they have refractive indices close to the refractive indices of gelling agent-liquid (including water and/or humectant) systems commonly used in dentifrices. In clear gels where the refractive index is an important consideration, 3—30% by weight of water, 0 to 80% by weight of glycerin, and 20—80% by weight of sorbitol is preferably employed. A gelling agent, such as a natural or synthetic gum or gum-like material, typically Irish moss, sodium carboxymethylcellulose, methyl cellulose, hydroxyethylcellulose, gum tragacanth, polyvinylpyrrolidone, starch, or preferably hydroxypropyl methyl cellulose or the Carbopols® (e.g. 934, 940 and 941) is usually present in toothpastes in an amount up to 10% by weight, preferably in the range of from 0.5 to 5%.

In a toothpaste or gel, the liquids and solids are proportioned to form a creamy or gelled mass which is extrudable from a pressurized container or from a collapsible, e.g. aluminum or lead, tube.

As noted previously, aqueous solutions of the zinc and fluoride components may be utilized in formulating other oral compositions wherein little or no moisture is present in the final product. For example, a chewing gum suitable for use as an oral vehicle herein comprises a gum base and flavoring materials such as those mentioned above for dentifrices. The flavoring materials are present at a level of 0.01% to 2.0% of the final chewing gum composition. The gum base is a chewable plastic gum material such as natural rubber, chicle, polyvinyl acetate, ester gum, coumarone resin, and paraffin wax. The gum base is typically made from a mixture of two or more plastic gum materials to achieve a preferred degree of plasticity for chewing. Corn syrup is generally added as a softener and binder for the chewing gum and sugar is optionally added as a filler and sweetener. A typical chewing gum suitable as a carrier herein comprises 15% to 30% gum base, 15% to 20% corn syrup, 50% to 65% sugar, and 0.05% to 1.5% flavoring materials. An aqueous solution of the zinc and fluoride components may be incorporated into the corn syrup prior to admixture with the gum base.

Lozenges suitable as carriers herein comprise a hard sugar candy base and one or more flavoring materials. The flavoring materials are present at levels between 0.01 to 2.0%. Optionally, lozenges can contain various other materials. A typical lozenge suitable as an oral vehicle in this invention is a hard candy comprised of a hard candy base containing 0.05% to 1.5% flavor. The hard candy base is a solidified

4

solution of amorphous sugar which is generally formed from a sugar solution which has been cooked at high temperature so as to remove nearly all of the moisture. The flavoring materials and the aqueous zinc/fluoride solution are added before the moisture is removed. The flavoring materials mentioned hereinbefore for dentifrices are also exemplary of those suitable for use in lozenges.

Mouth odor has been attributed to the presence of volatile sulfur compounds (VSC) such as hydrogen sulfide, methyl mercaptan and dimethyl sulfide resulting from putrefractive processes, occuring in the oral cavity. An instrumental gas chromatography (GC)—flame photometric detector (FPD) system is available to detect and measure sub-nanogram VSC levels in mouth air (see J. Tonzetich: Archs oral Biol., Vol. 16, pp. 587—597, 1971; and Intl. Dental J., Vol. 28, No. 3, pp. 309—319, 1978).

Utilizing such methodology, particularly effective results in terms of VSC (mouth odor) inhibition are obtained with the present oral compositions.

The following examples are further illustrative of the present invention, but it is to be understood that the invention is not limited thereto. All percents or parts in the examples are by weight unless otherwise specified.

### Examples 1—Chewing gum

| Component | Weight % |
|---|---|
| Zinc gluconate | 0.50 |
| Stannous fluoride | 0.10 |
| Distilled water | 2.50 |
| Gum base | 26.00 |
| Sucrose | 52.00 |
| Corn syrup | 17.00 |
| Flavor | 1.90 |

The zinc and fluoride salts are dissolved in the water (heated slightly) and the aqueous solution admixed with the corn syrup prior to incorporation with the other components.

### Examples 2—Lozenge

| Component | Weight % |
|---|---|
| Zinc salicylate | 0.50 |
| Ammonium fluoride | 0.10 |
| Sugar | 80.00 |
| Corn syrup | 11.00 |
| Flavor oil | 0.65 |
| Color | 0.25 |
| Tragacanth mucilage | 5.00 |
| Distilled water | 2.00 |

The zinc and fluoride salts are dissolved in the water (heated slightly) and the aqueous solution admixed with the corn syrup prior to incorporation with the other ingredients into a tenacious lozenge mass with sufficient plasticity for molding and cutting into desired shapes and sizes prior to drying.

**Claims**

1. A stable water-containing oral composition being essentially free of $ZnF_2$ precipitant comprising an effective mouth door inhibiting amount of an organic zinc salt selected from zinc salicylate, zinc lactate, zinc gluconate and mixtures thereof, an effective anti-caries amount of a water-soluble inorganic ionic fluoride salt, and being substantially free of additional adjuvants which inhibit the interaction between zinc and fluoride, and a semi-solid dentifrice vehicle containing at least sufficient water to solubilize said zinc salt and said fluoride salt.

2. The composition of claim 1 wherein said zinc salt is zinc salicylate.

3. The composition of claim 1 wherein said fluoride salt is a member selected from ammonium fluoride, an alkali metal fluoride and tin fluoride.

4. The composition of claim 1 wherein said zinc salt is zinc salicylate and said fluoride is sodium fluoride.

5. The composition of claim 1 wherein said composition is a dental cream, paste or gel.

6. The composition of claim 1 wherein said composition is a toothpaste.

**Patentansprüche**

1. Stabiles, Wasser enthaltendes orales Präparat, das im wesentlichen frei ist von $ZnF_2$-Fällungsmittel, enthaltend eine wirksame Menge eines Mundgeruch hemmenden organischen Zinksalzes, ausgewält aus Zinksalicylat, Zinklactat, Zinkgluconat und deren Gemischen und eine wirksame Menge eines als Karies-Schutzmittel wirkenden wasserlöslichen anorganischen ionischen Fluoridsalzes, wobei das Präparat im wesentlichen frei ist von zusätzlichen, die Wechselwirkung zwischen Zink und Fluorid hemmenden Hilfsstoffen und einen halbfesten Zahnputzmittel-Trägerstoff enthält, der wenigstens so viel Wasser aufweist, um das Zinksalz und das Fluoridsalz löslich zu machen.

2. Präparat nach Anspruch 1, in dem das Zinksalz Zinksalicylat ist.

3. Präparat nach Anspruch 1, in dem das Fluoridsalz ausgewählt ist aus Ammoniumfluorid, einem Alkalimetallfluorid und Zinnfluorid.

4. Präparat nach Anspruch 1, in dem das Zinksalz Zinksalicylat und das Fluorid Natriumfluorid ist.

5. Präparat nach Anspruch 1, in dem das Präparat eine Zahncreme, Zahnpasta oder ein Zahngel ist.

6. Präparat nach Anspruch 1, in dem das Präparat eine Zahnpasta ist.

**Revendications**

1. Une composition aqueuse stable pour l'usage oral, essentiellement exempte de précipité de $ZnF_2$, comprenant une quantité efficace à l'égard de la protection contre la mauvaise haleine d'un sel organique de zinc choisi parmi le salicylate de zinc, le lactate de zinc, le gluconate de zinc et leurs mélanges, une quantité efficace pour la protection contre les caries d'un fluorure minéral soluble dans l'eau inorganique et ionisé, et essentiellement exempte d'autres adjuvants inhibant l'interaction entre le zinc et le fluorure, et un véhicule semi-solide pour produits dentifrices contenant de l'eau en quantité au moins suffisante pour solubiliser le sel de zinc et de fluorure.

2. La composition de la revendication 1 dans laquelle le sel de zinc est le salicylate de zinc.

3. La composition de la revendication 1 dans laquelle le fluorure est choisi parmi le fluorure d'ammonium, les fluorures de métaux alcalins et le fluorure d'étain.

4. La composition de la revendication 1 dans lequelle le sel de zinc est le salicylate de zinc et le fluorure est le fluorure de sodium.

5. La composition de la revendication 1, qui est une crème dentifrice, une pâte dentifrice ou un gel dentifrice.

6. La composition de la revendication 1, qui est une pâte dentifrice.